# EUROPEAN PATENT SPECIFICATION

(11) **EP 1 608 755 B1**
(45) Date of publication and mention of the grant of the patent: **17.10.2012**
(21) Application number: 04724098.1
(22) Date of filing: 29.03.2004
(51) Int. Cl.: C12N 15/11, C12N 15/85, C12N 15/86, A61K 48/00

(54) **TAK1-MEDIATED INHIBITION OF OSTEOGENESIS**
TAK1-VERMITTELTE HEMMUNG DER OSTEOGENESE
BLOCAGE DE L'OSTEOGENESE PAR MEDIATION DU TAK-1

(30) Priority: 01.04.2003 US 458954 P
(43) Date of publication of application: 28.12.2005
(73) Proprietor: Helmholtz-Zentrum für Infektionsforschung GmbH, 38124 Braunschweig (DE)
(72) Inventor: Gazit, Dan, Jerusalem 96920 (IL); Pelled, Gadi, Rishon Lezion, 75424 (IL); Turgeman, Gadi, Jerusalem 97763 (IL); Hoffmann, Andrea, 30655 Hannover (DE); Gross, Gerhard, 38102 Braunschweig (DE); Wodarczyk, Claas, 38106 Braunschweig (DE); Verschueren, Kristin, 3078 Everberg (BE)
(74) Representative: Kröncke, Rolf
(86) International application number: PCT/IL2004/000286
(87) International publication number: WO 2004/087862

(56) References cited:
- EP-A- 1 234 880
- WO-A-99/40202
- JP-A- 9 163 990
- SUZAWA MIYUKI ET AL: "Cytokines suppress adipogenesis and PPAR-gamma function through the TAK1/TAB1/NIK cascade." NATURE CELL BIOLOGY, vol. 5, no. 3, March 2003 (2003-03), pages 224-230, XP002401218 ISSN: 1465-7392
- KIMURA N ET AL: "BMP2-induced apoptosis is mediated by activation of the TAK1-p38 Kinase pathway that is negatively regulated by SMAD6" JOURNAL OF BIOLOGICAL CHEMISTRY, THE AMERICAN SOCIETY OF BIOLOGICAL CHEMISTS, INC.,, US, vol. 275, no. 23, 9 June 2000 (2000-06-09), pages 17647-17652, XP002387983 ISSN: 0021-9258
- SHIBUYA ET AL: 'Role of TAK1 and TAB1 in BMP signaling in early Xenopus development' EMBO J vol. 17, no. 4, February 1998, pages 1019 - 1028, XP002988970
- MOCHIDA Y. ET AL: 'ASK1 Inhibits Interleukin-1-induced NF-B Activity through Disruption of TRAF6-TAK1 Interaction' J BIOL CHEM vol. 275, no. 42, October 2000, pages 32747 - 32752, XP002968451
- YOSHIDA Y. ET AL: 'Negative Regulation of BMP/Smad Signaling by Tob in Osteoblasts' CELL vol. 103, December 2000, pages 1085 - 1097, XP002988971
- CARNICI P. ET AL: 'Normalization and Subtraction of Cap-Trapper-Selected cDNAs to Prepare Full-Length cDNA Libraries for Rapid Discovery of New Genes' GENOME RES vol. 10, no. 10, October 2000, pages 1617 - 1630, XP002944079
- YAMAGUCHI K. ET AL: 'Identification of a member of the MAPKKK family as a potential mediator of TGF-beta signal transduction' SCIENCE vol. 270, 22 December 1995, pages 2008 - 2011, XP002103399
- HOFFMAN A. ET AL: 'Transforming Growth Factor-beta-activated Kinase-1 (TAK1), a MAP3K, Interacts with Smad Proteins and Interferes with Osteogenesis in Murine Mesenchymal Progenitors' J BIOL CHEM, [Online] vol. 29, May 2005, pages 1 - 33, XP008050479 Retrieved from the Internet: <URL:http://www.jbc.org/cgi/content/abstrac t/M503368200v1>

## Description

### FIELD OF THE INVENTION

The present invention is directed to the use of nucleic acids in TAKI-mediated regulation of SMAD activity, in particular, for promoting osteogenesis.

### BACKGROUND OF THE INVENTION

TGF-ss activated kinase (TAK1) was initially identified as a cytosolic component of mitogen activated protein kinase (MAP) pathways activated by ligands of the TGF-P and BMP family of secreted factors. TAK1 is a MAP kinase kinase kinase (MAPKKK, MAP3K) activated by the cytokines IL-1 and TNF-a, in addition to TGF-P/BMPs, consisting of roughly 600 amino acids, with an N-terminal kinase-domain of roughly 300 amino acids, which is roughly 30 % homologous to catalytic domains of other MAP3Ks (e. g. Raf-1, MEKK-1). TAK1 mediates activation of c-jun N-terminai kinases (JNK), p3 8-MAPK and NF-KB pathways. However, molecular events in TAK1 involved signaling cascades, in particular early events in the cascade are as yet not well defined.

SMAD proteins are a family of eight intracellular proteins (SMAD1 to SMAD8) whose members transduce signals for TGF-P ligands including the bone morphogenetic proteins (BMPs). SMAD proteins can be classified into three types according to their structure and mechanism of action. The receptor-regulated SMADs (R-SMADs) SMAD 1-3, SMAD5 and SMAD8, are directly phosphorylated and activated by a TGF-ss/BMP type I receptor. Another SMAD type, the common mediator SMAD (Co-SMAD; SMAD4), associates with activated R-SMADs, although hetero-oligomeric activated R-SMAD complexes without SMAD4 have also been detected. SMAD complexes accumulate in the nucleus and participate in the regulation of target genes. Regulation is predominantly at the level of complex binding to other transcription factors, and transcriptional regulators, however, direct SMAD binding to DNA via SMAD MH1 domain, or a combination of both mechanisms, may participate in the regulation of target gene expression. The third type of SMADs (I-SMADs, SMAD6 and SMAD7) are termed anti-SMADs or inhibitory SMADs owing to their interference with the activation of, and subsequent complex formation by R-SMADs.

### SUMMARY OF THE INVENTION

This invention relates to nucleic acids in TAK1- mediated regulation of SMAD activity as defined in the claims.

In one embodiment, the invention provides a method of a method of diminishing or abrogating SMAD activity comprising the steps of contacting a cell with an agent that stimulates or enhances TAK1 expression, wherein TAK1 interacts with an MH2 domain of a SMAD protein, thereby diminishing or abrogating SMAD activity.

In another embodiment, the invention provides a method of stimulating or enhancing SMAD activity comprising the steps of contacting a cell with an agent that diminishes or abrogates TAK1 interaction with an MH2 domain of a SMAD protein, thereby stimulating or enhancing SMAD activity.

In another embodiment, the invention provides a method of stimulating or enhancing BMP-mediated SMAD activity comprising the steps of contacting a cell with an agent that diminishes or abrogates TAK 1 expression or function.

In another embodiment, the invention provides a method of diminishing or abrogating BMP-mediated SMAD activity comprising the steps of contacting a cell with an agent that stimulates or enhances TAK1 expression or function.

The invention provides agents for use in enhancing osteogenesis in a subject in need said agent mitigates or abrogates TAK1 expression or function, thereby enhancing osteogenesis in said subject.

The invention provides agents for use in enhancing bone repair in a body of a subject in need said agent mitigates or abrogates TAK1 expression or function, thereby enhancing bone repair in a body of said subject.

### BRIEF DESCRIPTION OF THE DRAWINGS

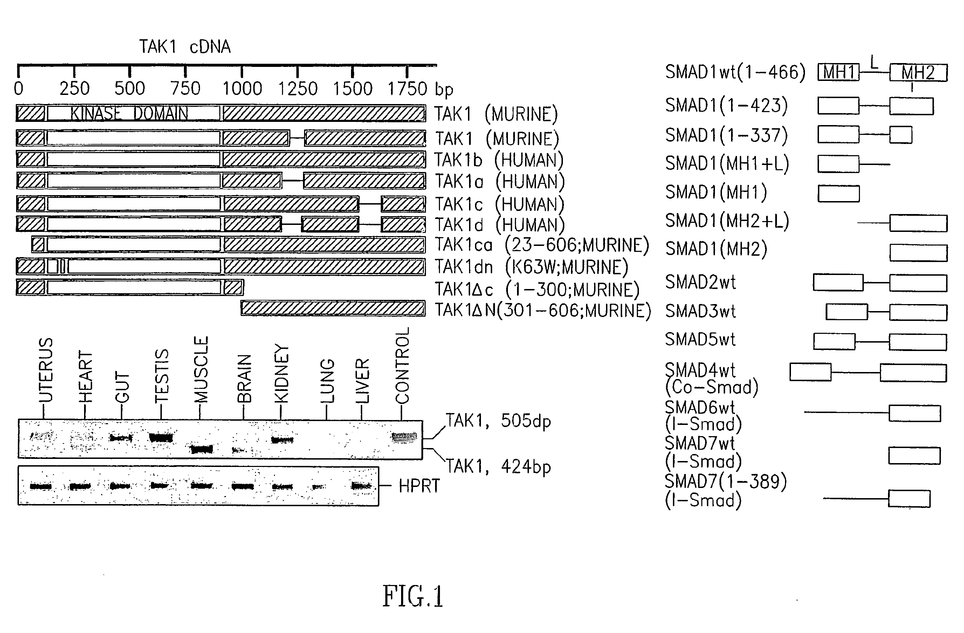

Figure 1A depicts the full-length murine TAK1 cloned by PCR from total kidney RNA. Murine TAK1 cDNA clones corresponded to the full-length human TAK1b sequence representing an unspliced TAK1 variant, (Genbank accession number: XM~131329). TAK1 splice variants, kinase domain location, TAK1 deletions, constitutively active (ca) and dominant negative (dn) variants used are as illustrated.
Figure 1B demonstrates the tissue distribution of the two TAK1 splice variant mRIA in mouse tissue as detected, by RT-PCR.
Figure 1C is a schematic representation of wild-type SMADs and SMAD1 domains used for functional studies in HEK293T and murine C3HIOT2 cells. MH1 and MH2 are the major conserved SMAD-domains while"L"specifies the linker region located between MH1 and MH2 domains.

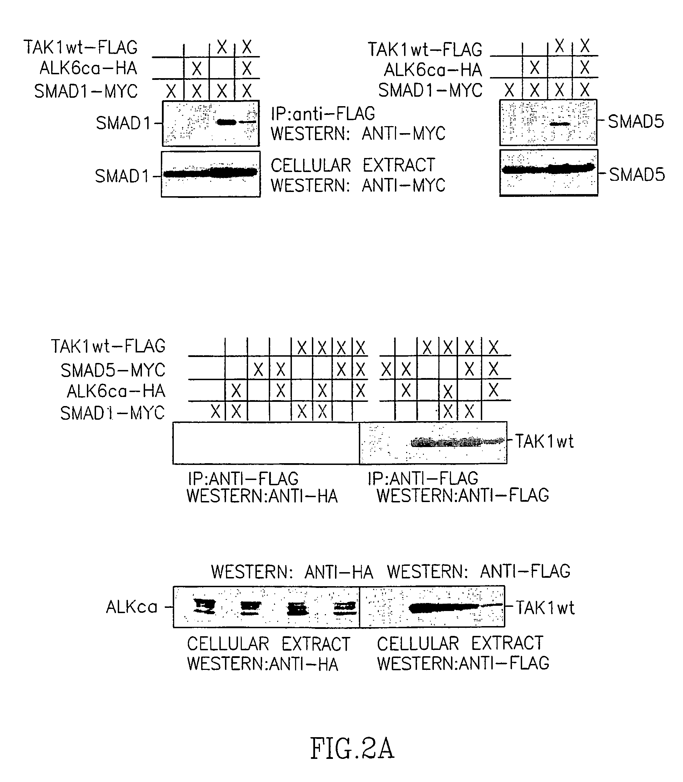

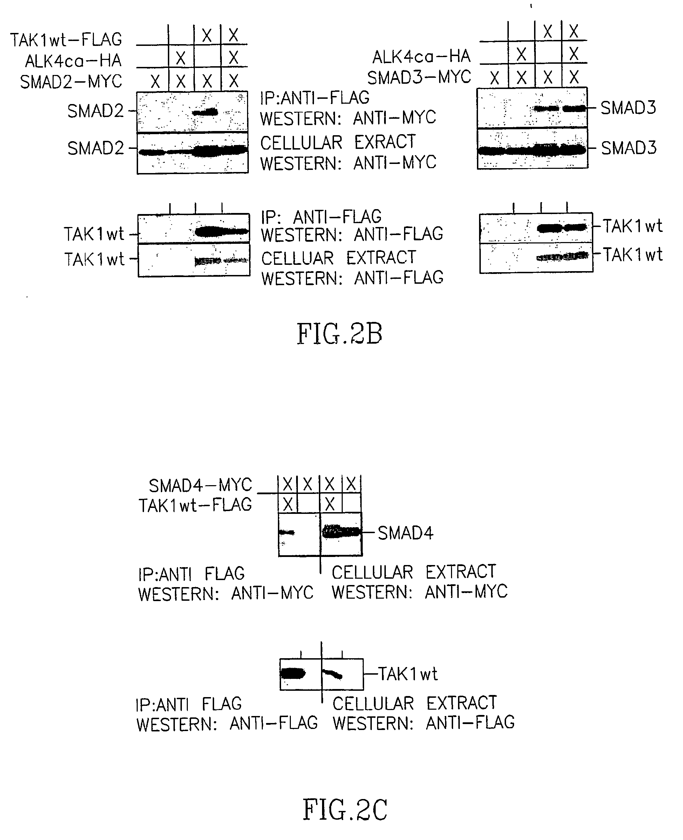

Figure 2 demonstrates TAK1 interaction with R-SMAD1, SMAD5, and with SMAD4. Human embryonic kidney (HEK) 293T cells were transfected with Flag-, Myc-or HA-tagged expression plasmids. The total amount of DNA transfected was adjusted with empty vector where appropriate. After transfection the cells were harvested, lysed and subjected to immunoprecipitation (IP). Cell extracts and immunoprecipitates were analyzed by SDS-PAGE followed by Western analysis, using the appropriate anti-tag-specific antibodies. SMAD-1 and SMAD-5 co- immunoprecipitated with TAK1 (A). SMAD-2, SMAD-3 (B) and SMAD-4 (C) similarly associated with TAK1.

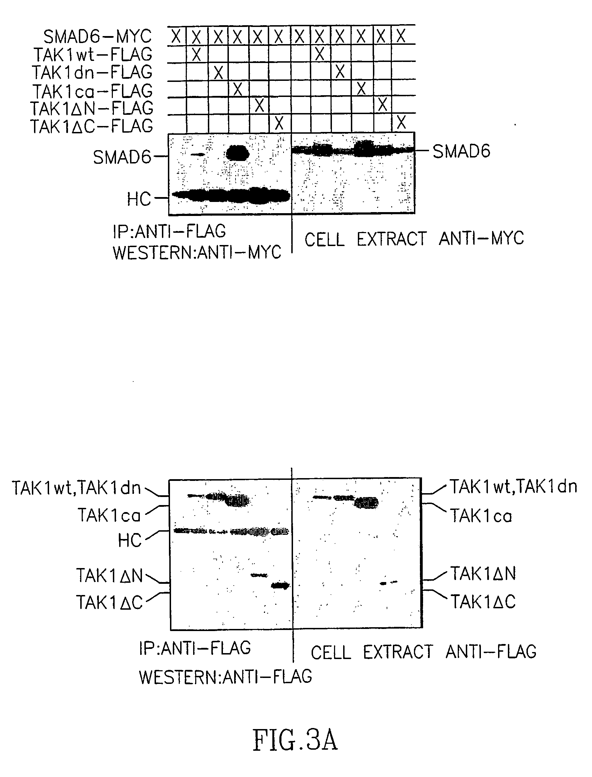

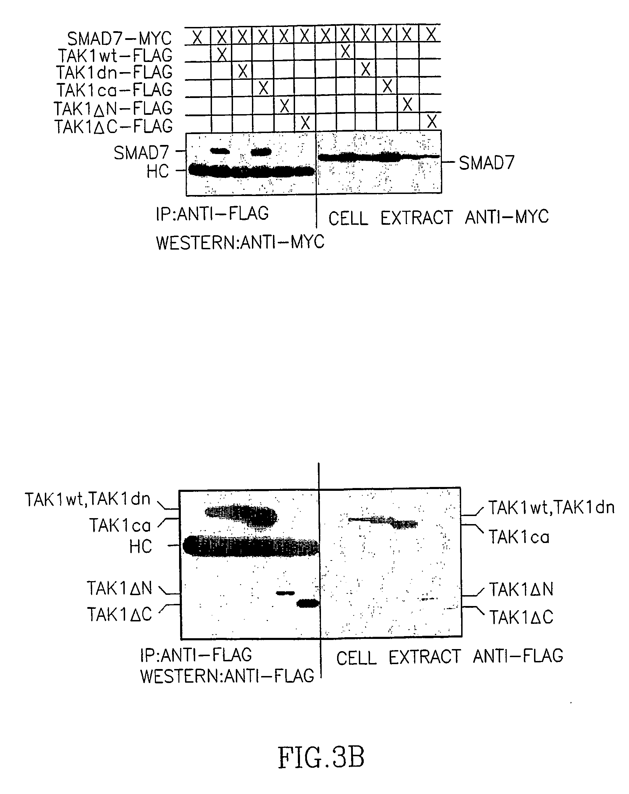

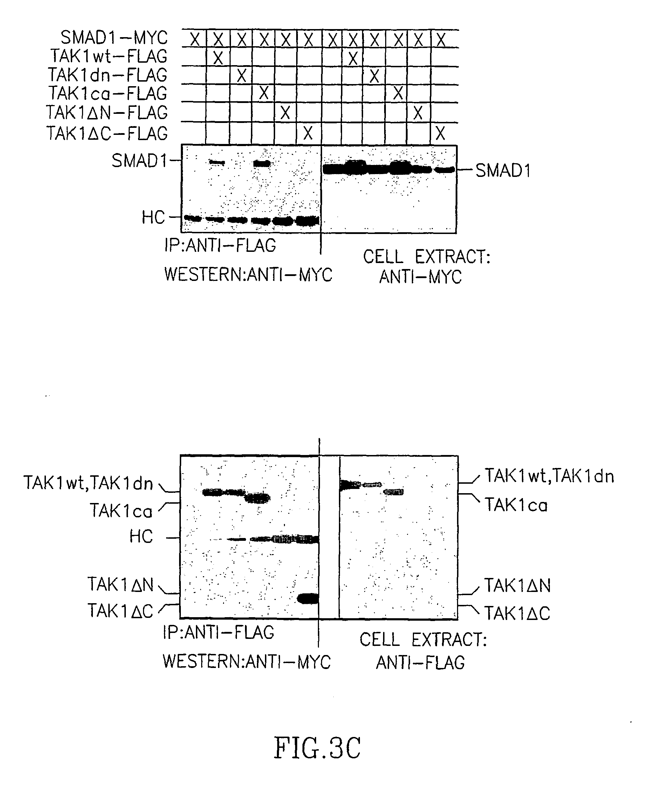

Figure 3 demonstrates that TAK1 interaction with R-SMAD1 or with I-SMAD6 (and SMAD7) was dependent on the presence of an active kinase domain within TAK1.
   Human embryonic kidney (HEK) 293T cells were transfected with Flag-or Myc-tagged expression plasmids and cell extracts were processed as in Figure 2. SMAD6 (panel (A)) and SMAD-7 (panel (B)) associated with TAK1 containing an active kinase domain, in non-stimulated cells. (C) SMAD1 also interacted only with TAK1 harboring an intact kinase domain. Deletions in TAK1 (1-300 (DC) and 301-606 (DN)) affected SMAD binding, indicating that both the TAK1 kinase domain and the remaining moiety were necessary for SMAD interaction.

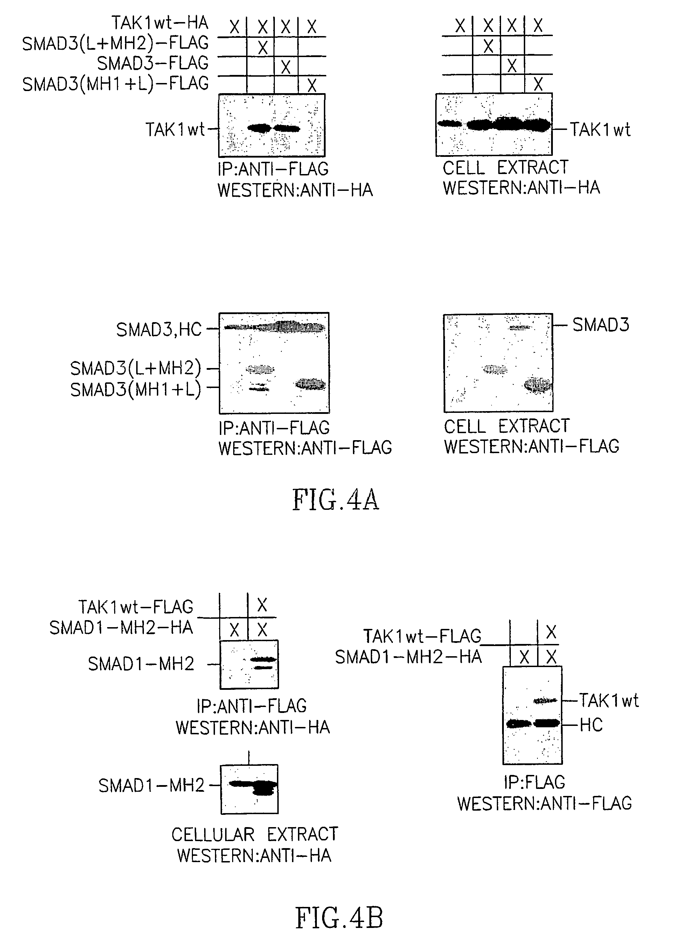

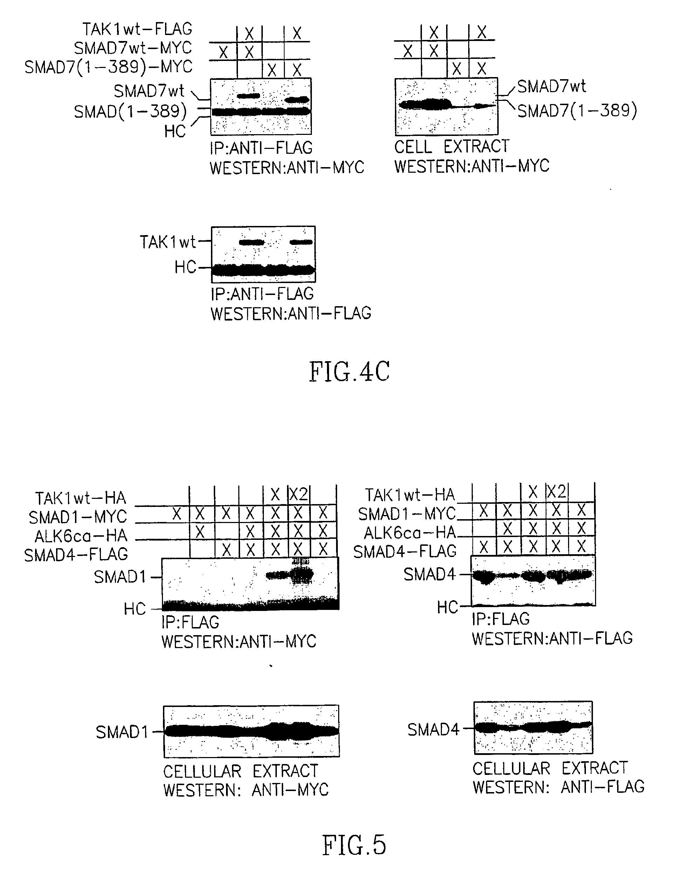

Figure 4 demonstrates that SMAD interaction with TAK1 was mediated via a conserved SMAD-MH2 domain. Human embryonic kidney (HEK) 293T cells were transfected with Flag-or Myc-tagged expression plasmids, and cell extracts were treated as described in the legend to Fig. 2. SMAD3 interaction with TAK1 occurs via the MH2 domain of the SMAD protein (A). The SMAD1-MH2 domain was sufficient for TAK1 interaction (B). A 38 amino acid deletion in the carboxy-terminal domain of the SMAD7-MH2 domain (SMAD7 (1-389) mutant) still associated with TAK1, demonstrating that the TAK1 binding domain is within a region of about 160 amino acids (C).
Figure 5 demonstrates that TAK1, activity stimulated a SMAD1 : SMAD4 interaction. Human embryonic kidney (HEK) 293T cells were transfected with HA-or Myc-tagged expression plasmids and cell extracts were processed as described in Figure 2. X2 indicates that twice the amount of TAK1wt expression vector was added. The SMAD1 : SMAD4 interaction was enhanced in the presence of TAKlwt.

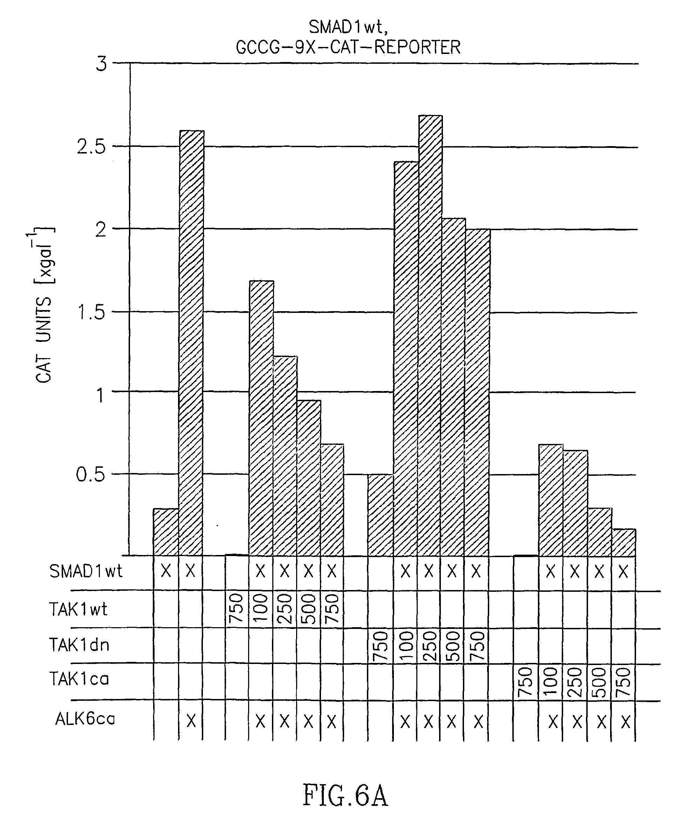

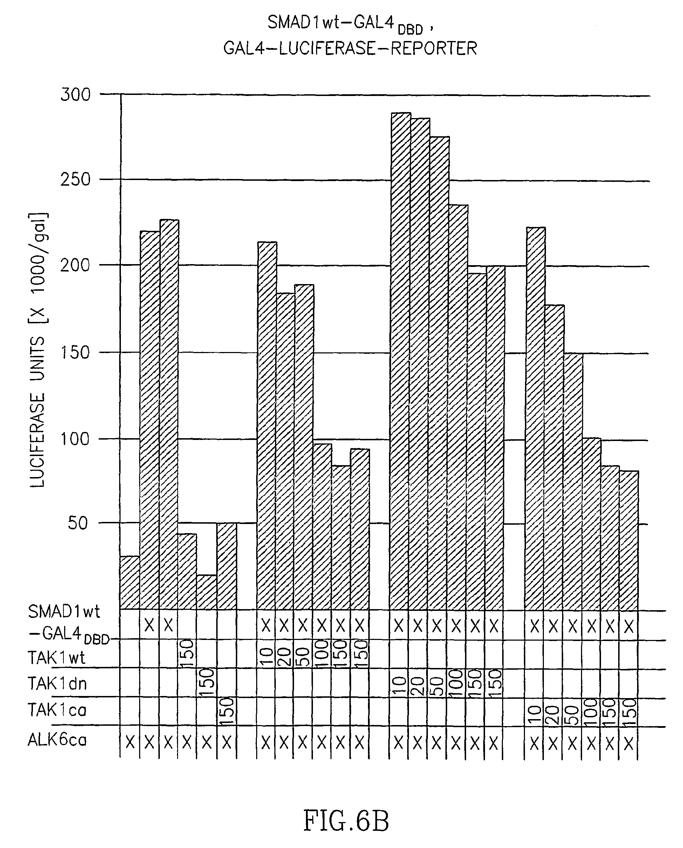

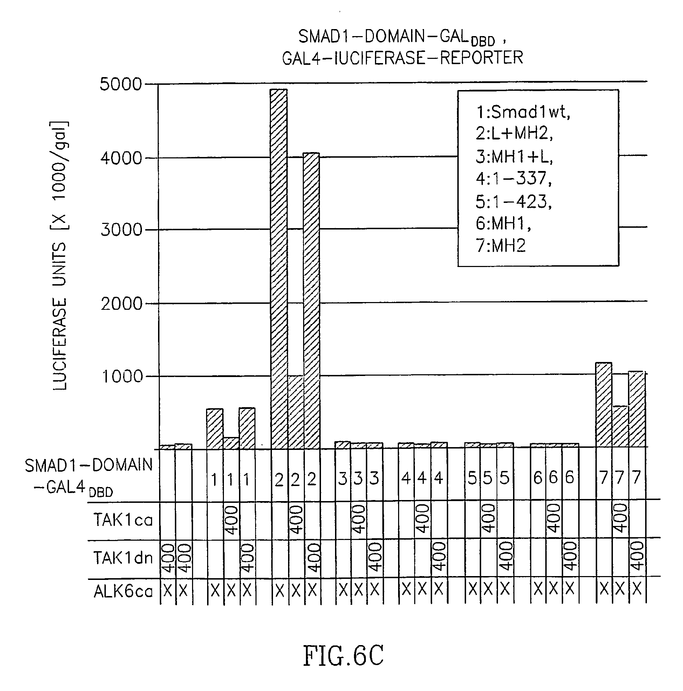

Figure 6 demonstrates that TAK1 negatively interferes with the transactivation potential of the SMAD-MH2 domain. Reporter assays using the SMAD1-SBE with full- length SMAD1 (A) or a GAL4 reporter with the GAL4 DNA binding (GAL4DBD) domain fused to various forms of SMAD proteins (B-C), in the presence of TAK1 variants, were performed as described. The SBE reporter encodes the CAT gene under the control of nine SMAD1-SBEs, while the GAL4 reporter plasmid encodes firefly luciferase under the control of five UAS sites upstream of a minimal promoter. Results are expressed as CAT or luciferase units normalized to b-galactosidase activity (which results from co-transfected RSV-LTR promoter-E. coli LacZ plasmid) o TAK1 interfered with SMAD1-mediated transcription in a dose-dependent fashion (A). TAK1 interfered with the SMAD1-dependent transactivation potential in a dose-dependent fashion (B).
   TAK1 mediated interference with SMAD1 transactivation required the presence of a complete SMAD-MH2-domain (C). Numbers for TAK1-variants reflect the amount of expression vector added (in nanograms).

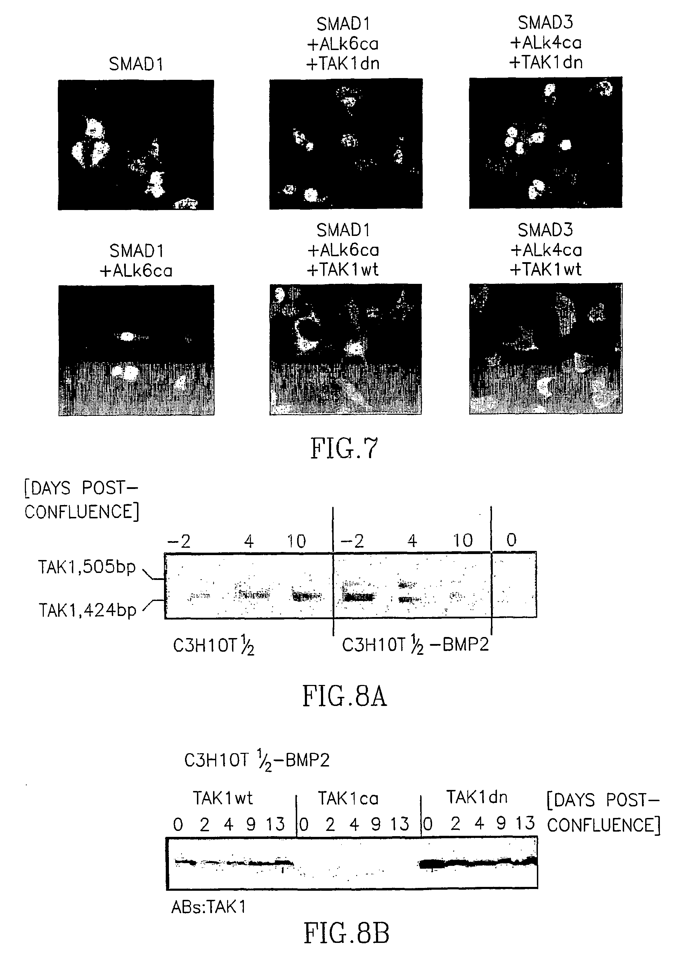

Figure 7 demonstrates TAK1 interference with nucleo-cytoplasmic shuttling of SMADs. Flag-tagged SMAD1 and SMAD3 were visualized by indirect immunofluorescence using an anti-Flag antibody after transfection of the appropriate expression vectors into HEK293T cells Active TAK1 interfered with nucleo-cytoplamic shuttling as evidenced by SMAD retention within the cytoplasm.

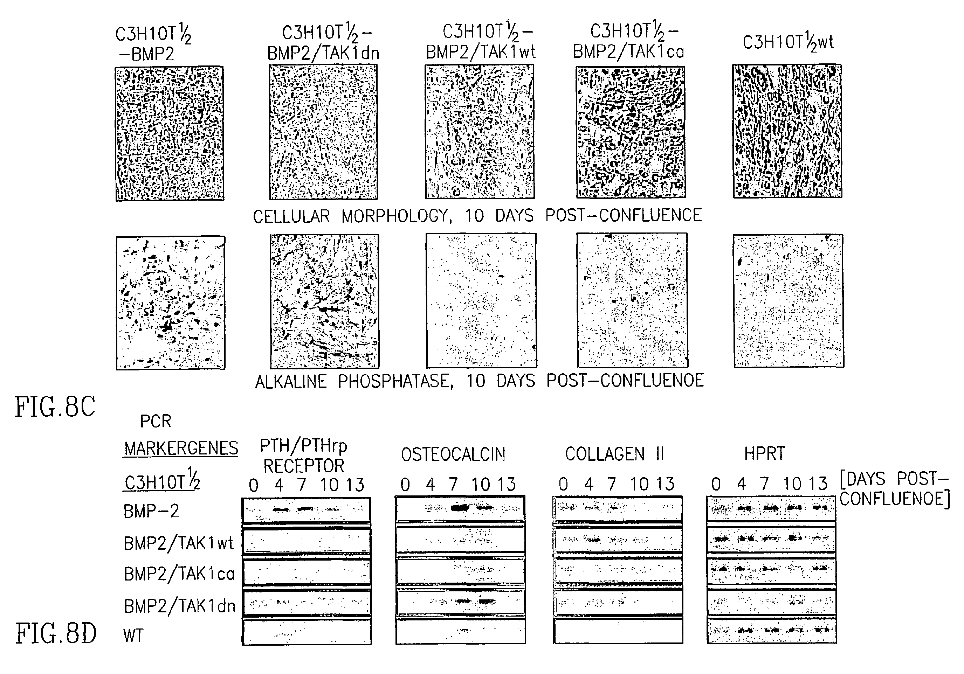

Figure 8 demonstrates TAK1 activity interfered with BMP-dependent differentiation potential of murine mesenchymal progenitors (C3HlOT'/2). Expression of a TAK1 mRNA splice-variant in murine mesenchymal progenitors C3HlOTi/2 was detected by PCR (A). Western, analysis using a polyclonal anti-TAKI antibody revealed expression levels of recombinantly expressed TAK1 proteins in stably transfected C3HlOT1/2-BMP2 cell lines, (endoge-nousTAK1 is not visualized under these conditions) (B). TAK1ca contains a deletion of 22 amino acids and is, therefore, smaller than wild- type (wt) and dominant-negative (dn ; W63K) TAK1 forms (arrows). The synthesis level for TAKlca is lower due to its cytotoxicity, allowing expression of low to moderate levels, only. Histological analysis of C3H1 OT1/2wt and C3H1OT1/2-BMP2 cells expressing TAIL1 variants at 10 days post-confluence (C). While C3HIOT'/2-BMP2 and C3H1OT1/2-BMP2/TAK1dn grow in multilayers (upper panels) C3H10T%- BMP2/TAKlca and C3H10T1/2-BMP2/TAK1wt cells grow in monolayer similar to C3H10T1/2wt (lower panels). C3H10T-BMP2/TAK1dn cells displayed enhanced osteoblast-like cell formation in comparison to C3H10T'/2-BMP2 cells, as evidenced by alkaline phosphatase staining. TAKlwt and TAK1ca interfered with osteoblast-like cell formation in C3HI1OT1/2-BMP2 cells. Semi-quantitative PCR analysis of osteo- /chondrogenic marker gene expression demonstrated reduced expression of osteogenic marker genes (PTH/PTHrP receptor; osteocalcin) but not chondrogenic marker genes (collagen II) in TAKlwt and TAKIca samples (D).

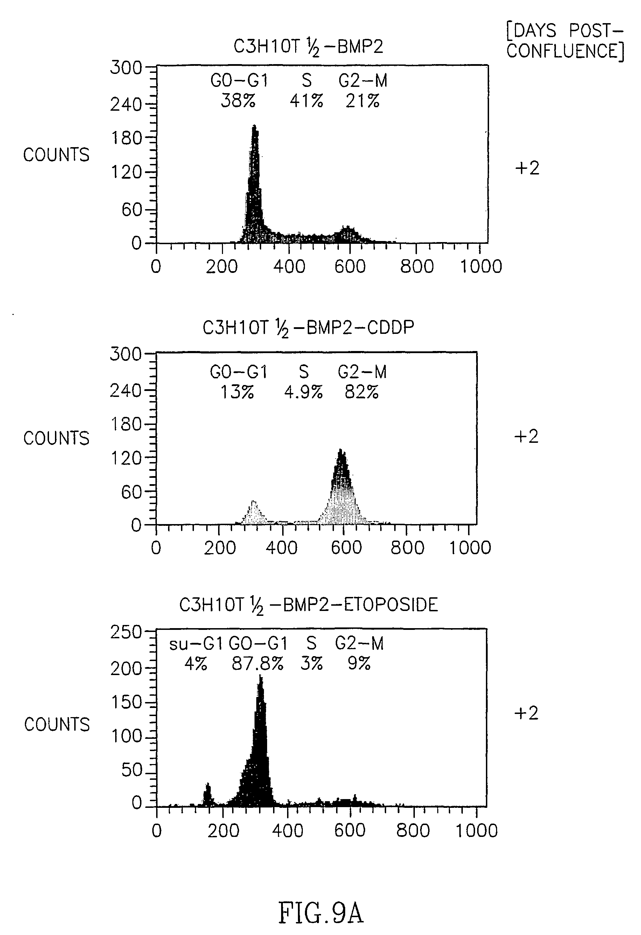

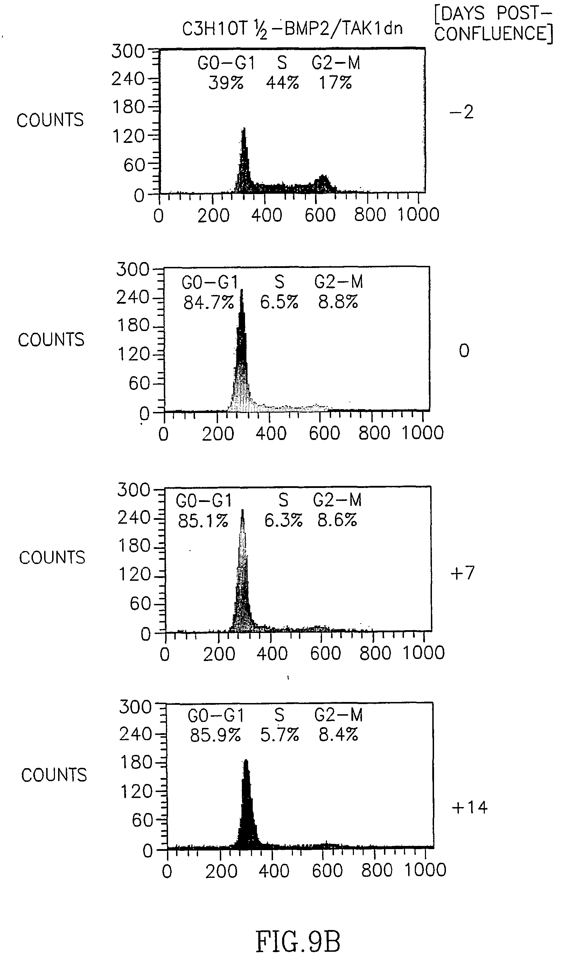

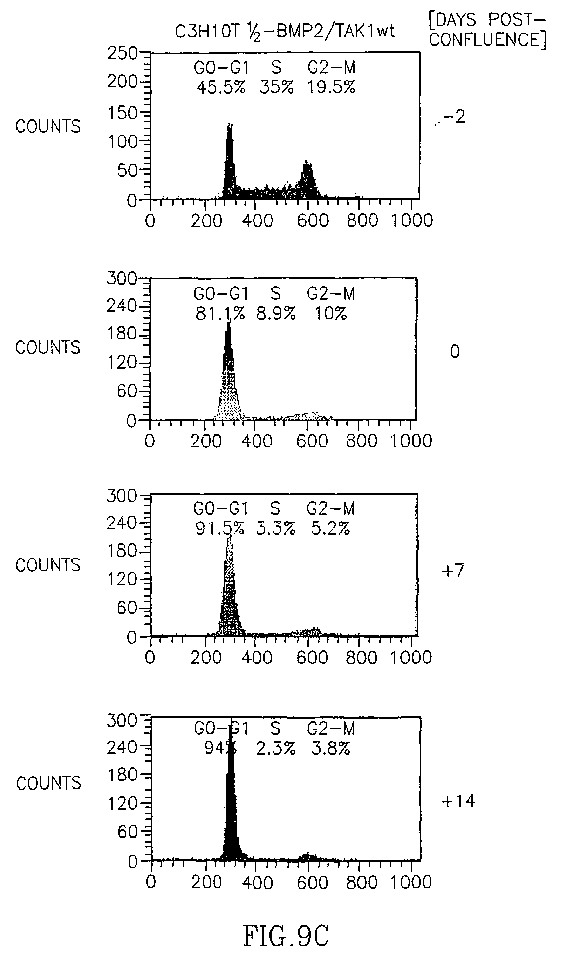

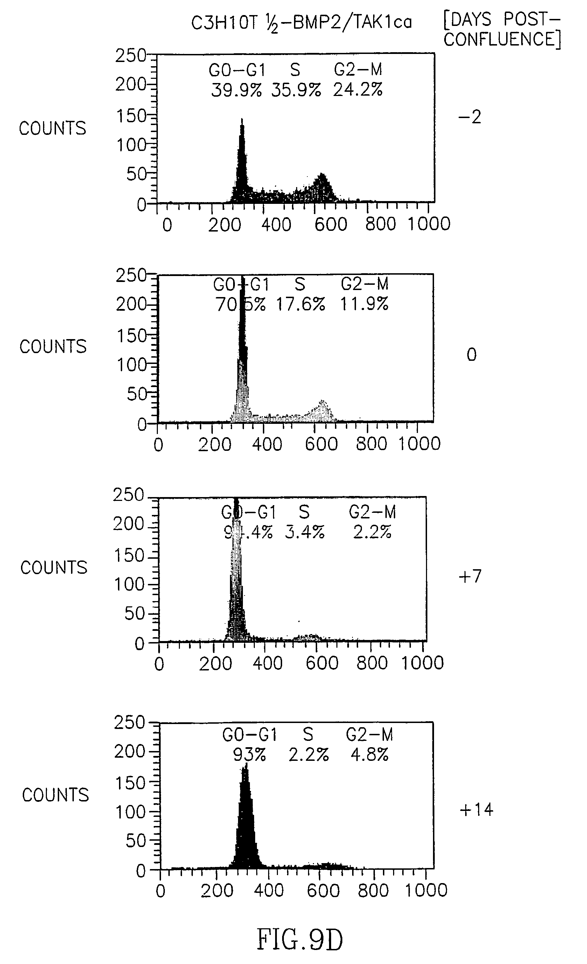

Figure 9 demonstrates TAK1 negligibly influences cell cycle progression in C3H10T'/z-BMP2 cells. Exponentially growing cells were harvested at indicated times prior to and following cellular confluence (confluence = day 0). C3H1OTy2-BMP2 cells in the presence of known apoptosis-inducing agent CDDP (25 u. M) arrested in G2-M, while the addition of known apoptosis-inducing agent etoposide (10 uM) caused measurable apoptosis, as visualized by a significant subGO-Gl peak. The shoulder in the G0-G1 peak indicated the presence of cells in early apoptosis, as well (arrows) (A). Dominant negative, wild-type and active TAK1 did not induce apoptosis in C3H10T1/2-BMP2 cells (C3H10T1/2-BMP2/TAK1dn, C3H10T1/2-BMP2/TAKlwt or C3H10T1/2- BMP2/TAK1ca) nor significantly change proportions of cells in GO/G1 versus G2/M phases (X-axis indicates relative DNA concentration; Y-axis indicates cell numbers, 3 independent experiments conducted, representative results shown) (B-D).

### DETAILED DESCRIPTION OF THE INVENTION

This invention provides nucleic acids for regulating SMAD expression or activity via TAK1, and therapeutic applications arising from their utilization as defined in the claims.

SMADs are a family of intracellular signaling proteins, which transduce signals thereby mediating a wide range of biological processes, including regulation of cell proliferation, differentiation, recognition, and death, and thus play a major role in developmental processes, tissue recycling, and repair. Effects that result in enhanced or diminished SMAD protein expression, or otherwise limit or enhance SMAD signal transduction will have substantial impact on biological processes.

TGF- activated kinase (TAlkl) is a MAP3K activated by ligands of the TGF-P and BMP family of secreted factors and by the cytoldnes IL-1 and TNF-a. Like the SMADs, TAK1 is involved in signaling cascades, with a definitive role in negative regulation of SMAD biological activity.

Direct interaction of TAK1 with SMADs is demonstrated in Example 2, hereinbelow, with intact TAK1 kinase activity being a prerequisite for SMAD binding. The conserved SMAD MH2 domain is the minimal domain required for the TAK1 interaction (Example 3).

TAK1 interaction with SMAD via its MH2 domain results in reduction of SMAD activity, in terms of its transactivation potential and transcription following receptor- mediated activation (Example 4). Thus, in its native form, TAK1 negatively-regulates SMAD biological activity.

By the term"biological activity"or"SMAD activity"or"SMAD, biological activity", it is meant, in one embodiment, to include all intracellular activity mediated via SMAD, including downstream effects in a given signal cascade, which are halted or altered as a result of SMAD'absence or diminution in concentration. Any function attributable to SMAD involvement is considered encompassed by the term.

The term"TAK1 "as used herein is meant, in one embodiment, to denote any TGF- activated kinase involved in signaling cascades, intracellularly, which in its native state interacts with a SMAD. In one embodiment, the TAK1 protein corresponds to, or in another embodiment is homologous to, Genbank Accession numbers 043318, NP~663304, NP~663305 or NP~663306, or is homologous to Genban1c Accession numbers NM~172688, NM 079356.

In another embodiment, the TAK1 protein is encoded by a nucleic acid sequence that corresponds to, or in another embodiment is homologous to, Genbanlc Accession numbers NM145333, NM145332, NM145331, NM003188 or is homologous to Genbank Accession number NP-524080.

As used herein, the term"correspond to"or"colTespondance"in reference to a protein or nucleic acid refers to an amino acid or nucleic acid sequence, respectively, that is identical to the referenced Sequence. The terms"homology","homologue"or "homologous", in any instance, indicate that the nucleic acid or amino acid sequence referred to, exhibits, in one embodiment at least 70 % correspondence with the indicated sequence. In another embodiment, the nucleic acid or amino acid sequence exhibits at least 75 % correspondence with the indicated sequence. In another embodiment, the nucleic acid or amino acid sequence exhibits at least 80 % correspondence with the indicated sequence. In another embodiment, the nucleic acid or amino acid sequence exhibits at least 85 % correspondence with the indicated sequence. In another embodiment, the nucleic acid or amino acid sequence exhibits at least 90 % correspondence with the indicated sequence. In another embodiment, the nucleic acid or amino acid sequence exhibits at least 95 % or more correspondence with the indicated sequence. In another embodiment, the nucleic acid or amino acid sequence exhibits 95 %-100 % correspondence with the indicated sequence.

Protein and/or peptide homology for any peptide sequence listed herein may be determined by immunoblot analysis, or via computer algorithm analysis of amino, acid sequences, utilizing any of a number of software packages available, via methods well known to one skilled in the art. Some of these packages may include the FASTA, BLAST, MPsrch or Scanps packages, and may employ the use of the Smith and Waterman algorithms, and/or global/local or BLOCKS alignments for analysis, for example.

Nucleic acid sequence homology may be determined by any number of computer algorithms available and well known to those skilled in the art, for example, the Smith-Waterman algorithm, utilized in analyzing sequence alignment protocols, as in for example, the GAP, BESTFIT, FASTA and TFASTA programs in the Wisconsin Genetics Software Package release 7.0, Genetics Computer Group, 575 Science Dr., Madison, WI). For example, the percent homology between two nucleotide sequences may be determined using the GAP program in the GCG software package, using a NWS gap DNA CMP matrix and a gap weight of 40,50, 60,70, or 80 and a length weight of 1, 2, 3, 4, 5, or 6.

Nucleic acid sequence homology may be determined by hybridization to a reference sequence under highly stringent (0.2xSSC at 65 °C.), stringent (e. g. 4xSSC at 65 °C or 50% formamide and 4xSSC at 42 °C.), or relaxed (4xSSC at 50 °C. or 30-40% formamide and 4xSSC at 42 °C.) conditions.

By the term"SMAD"or"SMADs"as used herein, it is meant to include, in one embodiment, any family member of the SMAD intracellular signaling proteins, which transduce signals for TGF- , regardless of species. SMADs include but are not limited to SMAD-1, SMAD-2, SMAD-3, SMAD-4, SMAD-5, SMAD-6, SMAD-7 or SMAD-8.

As contemplated herein, the nucleic acid, which encodes for a SMAD protein may correspond to, or in another embodiment, be homologous to Genbanlc Accession Numbers: NM 005905, NT 016606, NM 008539, AF 067727, NM 010754, AB 071949, AH006488, AF 056001, AB 008192, NM 005902, NM 016769, NT 010265, NT 033905, AB 043547, AB 010954, AF 056002, NT 016714, AH005750, AH 005612, MN 008541" AB043547, AH008461, AF037469, AF. 043640, AH011391, AH008243, AJ000550, AF175408, MN 139972, MN 005905 or MN 19483.

As contemplated herein, the SMAD amino acid sequence may correspond to, or in another embodiment, be homologous to, Genbank Accession Numbers: Q92940, Q17796, Q99717,043541, 035253, Q15797, BAA22032, AAB94137, S68987, AAC50790, AAB06852,015105 or Q15796. In another embodiment, the SMAD amino acid sequence may be homologous to Genbank Accession Numbers: AAN85445, JE0341, P97454, Q62432, P97588,070436.

The terms"diminishing/es","mitigating/es","down-regulating/es"or"down- modulating/es"are to be considered synonymous, and meant to include, in one embodiment, quantitative or qualitative reduction. Signaling molecules typically are active in exquisitely low intracellular concentrations, and hence minute alterations in these concentrations may result in a biologically observable effect.

By diminishing SMAD activity, we mean interference with SMAD intracellular function. Such interference may be via physical prevention of SMAD interaction with accessory proteins. Such inhibition of SMAD intracellular function may be carried out with minute amounts of TAK1.

SMAD proteins have been shown to function as transcriptional activators, and as such, two hybrid systems may be utilized to measure changes in SMAD transactivational activity, as a measure of SMAD function, as further exemplified in the Examples section that follows. SMAD activity may be measured as a function of its ability to translocate to the nucleus. This can be measured by any number of techniques known in the art, such as subcellular fractionation followed by immunoblot analysis of the fractions, or for example, by immunocytochemistry, as further exemplified in the Examples section that follows.

Diminishing SMAD activity may also include preventing SMAD expression. Receptor-mediated cellular activation, for example, resulting ordinarily in SMAD transcription may be affected by intracellular TAK1 expression, resulting in diminished SMAD transcription.

Changes in SMAD gene expression may be measured by methods well described in the art, such as Northern blot and dot blot analysis, primer extension, RNase protection, RT-PCR, or in-situ hybridization.

The terms 6abrogating"or"abrogation"refer to the absence of activity, including mRNA or protein expression.

By the term"stimulate/s"it is meant to include any effect which results in the initiation of production of the molecule/s specified, and can include events resulting in the initiation of mRNA expression, or protein production. The term"enhance/s"refers, in one embodiment, to a heightened, greater production of the molecule specified, including, as above, increased mRNA or protein production.

The term"an agent that stimulates or enhances TAK1 expression", includes any chemical entity that produces an increased expression of TAK1 mRNA or protein. Further, it includes a chemical entity that provides for the expression of TAK1 mRNA or protein, where previously expression of TAK1 was absent.

Increased production of TAK1 mRNA may be demonstrated via numerous methods well known to one skilled in the art. Some methods include Northern blot and dot blot analysis, primer extension, RNase protection, RT-PCR, or in-situ hybridization (see, for example"Current Protocols in Molecular Biology"Volumes I-III Ausubel, R. M., ed. (1994) ; Ausubel et al., !"Current Protocols in Molecular Biology", John Wiley and Sons, Baltimore, Maryland (1989) ; Mullis & Faloona, 1987, Methods Enzymol., 155: 335, Dassi et al. , 1998, Clin. Chem. , 44: 2416). In another embodiment, measurement of TAK1 protein production is another means of verifying TAK1 expression. Methods for detection, of TAK1 protein include Western blot analysis, immunoblot analysis, ELISA, RIA or HPLC, to name a few examples. Such an agent can comprise nucleic acids, or nucleic acid vectors comprising a TAK1 gene, that when introduced are expressed intracellularly, resulting in expression, or elevated expression of TAK1 mRNA and protein.

The term"nucleic acid"describes a polymer of deoxyribonucleotides (DNA) or ribonucleotides (RNA). The nucleic acid may be isolated from a natural source by cDNA cloning or subtractive hybridization or synthesized manually. The nucleic acid may be synthesized by chemical synthesis, manually by the triester synthetic method or by using an automated DNA synthesizer. The nucleic acid may be synthesized by in vitro amplification [including but not limited to the polymerase chain reaction (PCR)], or by combinations of these procedures from naturally occurring sources, such as cultures of mammalian cells, genomic DNA from such cells or libraries of such DNA. The nucleic acid may be single stranded, or double stranded and may assume any 3 dimensional structure. The nucleic acid sequence may also differ from the published TAK1 sequence, yet still encode for a TAK1 protein with an intact kinase domain, and is to be considered an additional embodiment of the present invention.

The terms"protein", "polypeptide"and"peptide"are used interchangeably herein when referring to a gene product.

As will be appreciated by one skilled in the art, a fragment or derivative of a nucleic acid sequence or gene that encodes for the TAK1 protein or peptide can still, in one embodiment, function in the same manner as the entire, wild type gene or sequence, in particular if it comprises the kinase domain of TAK1. Likewise, forms of nucleic acid sequences can have variations as compared with the wild type sequence, while the sequence still encodes a. protein, or peptide, or fragments thereof, that retain their wild type function despite these variations. Proteins, protein fragments, peptides, or derivatives also can experience deviations from the wild type from which still functioning in the same manner as the wild type form. Similarly, derivatives of the genes and products of interest used in the present invention will have the same biological effect on the host as the non-derivatized forms. Examples of such derivatives include but are not limited to dimerized or oligomerized forms of the genes or proteins, as wells as the genes or proteins. Biologically active derivatives and fragments of the genes, DNA sequences of the present invention are therefore also within the scope of this invention.

The agent may, in another embodiment, comprise a recombinant vector which comprises at least one nucleic acid sequence encoding the TAK1 protein or variant, analog, fragment, mimetic, mutant or synthetic thereof, and compositions comprising same. The nucleic acid sequence encoding the TAK1 protein or variant may be in single or multi-copy and may be conditionally or constitutively expressed, engineered by methods well known to one skilled in the art [see for example, "Molecular Cloning: A laboratory Manual"Sambrook et al. , (1989);"Current Protocols in Molecular Biology" Volumes 1-111 Ausubel, R. M., ed. (1994) ; Ausubel et al. 9"Current Protocols in Molecular Biology", John Wiley and Sons, Baltimore, Maryland (1989) ; Perbal, "A Practical Guide to Molecular Cloning", John Wiley & Sons, New York (1988) ; Watson et al.,"Recombinant DNA", Scientific American Books, New York].

Once TAK1 is subcloned into a particular vector it thereby becomes, in one embodiment, a recombinant vector. To generate the nucleic acid constructs in context of the present invention, the polynucleotide segments encoding TAK1 can be ligated into commercially available expression vector systems suitable for transfecting or transducing mammalian cells and for directing the expression of recombinant products within the transfected or transduced cells. It will be appreciated that such commercially available vector systems can easily be modified via commonly used recombinant techniques, in order to replace, duplicate or mutate existing promoter or enhancer sequences and/or introduce any additional polynucleotide sequences such as for example, sequences encoding additional selection markers or sequences encoding reporter polypeptides.

By"vector"what is meant is, in one embodiment, a nucleic acid construct containing a sequence of interest that has been subcloned within the vector, in this case, the nucleic acid sequence encoding TAK1 or a fragment thereof.

A vector according to the present invention may, according to additional embodiments, further include appropriate selectable markers. The vector may further include an origin of replication, and may be a shuttle vector, which can propagate both in prokaryotic, and in eukaryotic cells, or the vector may be constructed to facilitate its integration within the genome of an organism of choice. The vector according to this aspect of the present invention can be, for example, a plasmid, a bacmid, a phagemid, a cosmid, a phage, a virus or an artificial chromosome.

Protocols for producing recombinant vectors and for introducing the vectors into cells may be accomplished, for example, by: direct DNA uptake techniques, virus, plasmid, linear DNA or liposome mediated transduction, or transfection, magnetoporation methods employing calcium-phosphate mediated and IWAE-detran mediated methods of introduction, electroporation, direct injection, and receptor- mediated uptake (for further detail see, for example, "Methods in Enzymology"Vol. 1-317, Academic Press, Current Protocols in Molecular Biology, Ausubel F. M. et al. (eds.) Greene Publishing Associates, (1989) and in Molecular Cloning: A Laboratory Manual, 2nd Edition, Sambrook et al. Cold Spring Harbor Laboratory Press, (1989), or other standard laboratory manuals).

Additional suitable commercially available mammalian expression vectors include, but are not limited to, pcDNA3, pcDNA3. 1 (+/-), pZeoSV2 (+/-), pSecTag2, pDisplay, pEF/myc/cyto, pCMV/myc/cyto, pCR3. 1, which are available from Invitrogen, pCI which is available from Promega, pBK-RSV and pBK-CMV which are available from Stratagene, pTRES which is available from Clontech, and their derivatives. Linear DNA expression cassettes (LDNA) may be employed as well (Chen ZY et al Mol Ther. 3: 403- 1, 0, 2001).

In another embodiment, the vector is engineered to incorporate a reporter gene. The term"reporter gene", as used herein, refers to a coding unit whose product is easily assayed (such as, without limitation, luciferase or chloramphenicol transacetylase). A reporter gene can be either a DNA molecule isolated from genomic DNA, which may or may not contain introns, or a complementary DNA (cDNA) prepared using messenger RNA as a template. In either case, the DNA encodes an expression product that is readily measurable, e. g. , by biological activity assay, enzyme-linked immunosorbent assay (ELISA) or radioimmunoassay (RIA). Expression products of the reporter genes can be measured using standard methods. Various types of immunoassays such as competitive immunoassays, direct immunoassays and indirect immunoassays may be used as well, in order to detect the reporter gene product.

In addition to recombinant vectors enhancing TAK1 expression, other agents may be employed that function to stimulate TAK1 expression including factors that initiate the TGF-P signaling cascade, that result in production of TAK1, including but not limited to, TGF-P and TGF-P receptor agonists. Any agent that results in production or enhanced production of TAK1, which facilitates TAK1 binding to SMAD MH2 domains is considered to comprise additional embodiments of the present invention.

As used herein, the term"contacting a cell"refers, in one embodiment, to both direct and indirect exposure of a cell to an agent or molecule of the invention. In one embodiment, contacting a cell may comprise direct injection of the cell through any means well known in the art, such as microinjection. It is also envisaged, in another embodiment, that supply to the cell is indirect, such as via provision in a culture medium that surrounds the cell. Any agent of the invention may be administered thus, and comprises an embodiment of the invention.

As used herein, the term"administration""administer/ed","delivery"or "deliver/ed"refers" in one embodiment, to introduction that may be performed topically (including opthalmically, vaginally, rectally, intranasally), orally, by inhalation, or parent, erally, for example by intravenous drip or intraperitoneal, subcutaneous, subdural, intramuscular or intravenous injection, or via an implantable delivery device. Any agent of the invention may be administered thus, and comprises an embodiment of the invention.

It is to be understood that the agents of the invention may be administered, in one embodiment, as part of a pharmaceutical composition. Such a pharmaceutical composition may include the nucleic acids in combination with any standard physiologically and/or pharmaceutically acceptable carriers, which are known in the art. The compositions should be sterile and contain a therapeutically effective amount of the nucleic acids in a unit of weight or volume suitable for administration to a patient. The term"pharmaceutically acceptable"refers to a non-toxic material that does not interfere with the effectiveness of the biological activity of the active ingredients. The term "physiologically acceptable"refers to a non-toxic material that is compatible with a biological system such as a cell, cell culture, tissue, or organism. The characteristics of the carrier will depend on the route of administration. Physiologically and pharmaceutically acceptable carriers include diluents, fillers, salts, buffers, stabilizers, solubilizers, and other materials, which are well known in the art.

In another embodiment, the agent administered to stimulate or enhance TAK1 expression may comprise a mutated version of TAK1, which may be administered as a naked nucleic acid molecule, or within an expression vector. Mutations in TAK1 may enhance SMAD interaction, may function to promote uptake within cells, or may provide additional benefits for their introduction, and as such are considered as part of this invention. Mutations of the TAK1 nucleic acid sequence may comprise point mutations, substitutions, insertions or deletion mutations, or induced modifications each of which represent an additional embodiment of the invention. Nucleic Acid sequences of the present invention may comprise single mutations, or multiple mutations, including combinations of the mutations listed herein, each of which is to be considered a separate embodiment of the invention.

The mutations serve to enhance TAK1 mediated interaction with MH2 domains of SMAD, thereby negatively regulating SMAD activity.

The addition of low levels of a dominant-negative mutant of TAK1, however, not only reversed the suppressive effects on SMAD activity seen with wild-type and constitutively active TAK1, but enhanced transactivation and transcription (Example 4).

Dominant-negative (dn) mutations introduced into a gene result in loss-of-function for the encoded product, however, they typically retain their proper structure and associate with other cellular components, often with a higher than normal affinity for a cellular component, displacing the wild-type protein. In this case, the TAKldn mutant competed with endogenous TAK1.

Interfering with TAK1 function provides, in one embodiment, a means of stimulating or enhancing SMAD activity.

In another embodiment, there is provided a method of stimulating or enhancing SMAD activity comprising the steps of contacting a cell with an agent that diminishes or abrogates TAK1 interaction with an MH2 domain of a SMAD protein, thereby stimulating or enhancing SMAD activity.

By the phrase"diminishes or abrogates TAK1 interaction", it is meant to comprise an ultimate interference with the TAK1-SMAD MH2 interaction. Such an interference may be via a physical block of the MH2 SMAD domain, preventing TAK1 interaction, or a physical block of the cognate binding regions in TAK1, or according to the present invention a method of diminishing or abrogating TAK1 expression, thereby preventing or limiting its association with SMAD.

Down-regulation of endogenous sequences may be accomplished via various means well known in the art. In one embodiment, antisense RNA may be employed as a means of endogenous sequence inactivation. Exogenous polynucleotide (s) encoding sequences complementary to the endogenous TAK1 mRNA sequences, or fragments thereof are administered, resulting in sequence transcription, and subsequent gene inactivation.

In one embodiment, the TAK1 sequence targeted for gene inactivation corresponds to, or is homologous to SEQ ID No: 1 or 2.

In another embodiment, downregulating TAK1 gene expression is via the use of small interfering RNAs (siRNAs). Duplexes consisting of between 15-, and 30- nucleotide siRNA generated by ribonuclease III cleavage of longer dsRNAs, and by cleavage induced by other enzymes (e. g. ,"dicer"in D. melanogaster (Baulcombe, D. Nature 409 (2001): 295-6 and Caplen, N. J. , et al. PNAS. 98 (2001): 9742-7) thought to be similar to RNase III, or generated artificially, are the mediators of sequence specific mRNA degradation. Overhanging nucleotides on the 3'ends of the dsRNA help processing proteins recognize the dsRNA and mediate cleavage of the target mRNA. Duplexes associated with the processing protein form small interfering ribonucleoprotein complexes (siRNPs), possessing endonuclease activity, facilitate cutting of the nucleic acids. The siRNPs that contain antisense-siRNA hybridize to complementary sense mRNA and cleave it, and vice versa. Gene silencing entails antisense siRNA-mediated cleavage of target mRNA transcribed from the gene, preventing cells from translating the target mRNA into a protein (Hammond, SM et al Nature 404 : 293-296 (2000) ; Yang, D et al, Curr. Biol. 10: 1191-1200 (2000) ; and US Patent Application Serial No. 20020086356).

In another embodiment, disrupting TAK1 gene expression can be accomplished using synthetic oligonucleotides capable of hybridizing with endogenous TAK1 double stranded DNA. A triple helix is formed. Such oligonucleotides may prevent binding of transcription factors to the gene's promoter and therefore inhibit transcription. Alternatively, they may prevent duplex unwinding and, therefore, transcription of genes within the triple helical structure.

A dominant negative suppressor of TAK1 nucleotide sequence is at set forth in SEQ ID Nos : 1 or 2. In another embodiment, the nucleic acid sequence comprises a fragment thereof. TAIL1 nucleotide coordinates that may be inhibited, in one embodiment, from interacting with SMAD MH2 domains comprise nucleotides encoding for amino acids 43 to 284, which encodes the kinase domain of TAK1.

TAK1 deletion mutants comprise a nucleotide sequence as follows (SEQ ID No: 1) :

ATGTCGACAGCCTCCGCCGCCTCGTCCTCCTCCTCGTCTTCTGCCAGTGAGATGATCGAAGCGCCGTCGCAGGTCCTGA
ACTTCGAAGAGATCGACTACAAGGAGATCGAGGTGGAAGAGGTTGTCGGAAGAGGAGCTTTTGGAGTAGTTTGCAAAG
CTAAGTGGAGAGCAAAAGATGTCGCTATTAAACAGATAGAAAGTGAGTCTGAGAGGAAGGCTTTCATTGTGGAGCTCC
GGCAGTTGTCACGTGTGAACCATCCTAACATTGTCAAGTTGTATGGAGCCTGCCTGAATCCAGTATGTCTTGTGATGGA
ATATGCAGAGGGGGGCTCATTGTATAATGTGCTGCATGGTGCTGAACCATTGCCTTACTACACTGCTGCTCATGCCATG
AGCTGGTGTTTACAGTGTTCCCAAGGAGTGGCTTACCTGCACAGCATGCAGCCCAAAGCGCTGATTCACAGGGACCTC
AAGCCTCCAAACTTGCTGCTGGTTGCAGGAGGGACAGTTCTAAAAATCTGCGATTTTGGTACAGCTTGTGACATCCAA
ACACACATGACCAATAATAAAGGGAGTGCTGCTTGGATGGCGCCTGAAGTATTTGAAGGTAGCAATTACAGTGAAAAG
TGTGATGTCTTCAGCTGGGGTATTATCCTCTGGGAAGTGATAACACGCCGGAAACCCTTCGATGAGATCGGTGGCCCA
GCTTTCAGAATCATGTGGGCTGTTCATAATGGCACTCGACCACCACTGATCAAAAATTTACCTAAGCCCATTGAGAGCT
TGATGACACGCTGTTGGTCTAAGGACCCATCTCAGCGCCCTTCAATGGAGGAAATTGTGAAAATAATGACTCACTTGA

TGCGGTACTTCCCAGGAGCGGATGAGCCGTTACAGTATCCTTGTCAGTA

TAK1 deletion mutants comprise a nucleotide sequence as follows (SEQIDNo : 2):

CTCTGATGAAGGGCAGAGCAACTCAGCCACCAGCACAGGCTCATTCATGGACATTGCTTCTACAAATACCAGTAATAA
AAGTGACACAAATATGGAACAGGTTCCTGCCACAAACGACACTATTAAACGCTTGGAGTCAAAACTTTTGAAAAACCA
GGCAAAGCAACAGAGTGAATCTGGACGCCTGAGCTTGGGAGCCTCTCGTGGGAGCAGTGTGGAGAGCTTGCCCCCCA
CTTCCGAGGGCAAGAGGATGAGTGCTGACATGTCTGAAATAGAAGCCAGGATCGTGGCGACTGCAGCCTATTCCAAGC
CTAAACGGGGCCACCGTAAAACCGCTTCATTTGGCAACATTCTGGATGTCCCTGAGATCGTCATATCAGGTAACGGGC
AACCAAGGCGTAGATCCATCCAAGACTTGACTGTTACTGGACAGAACCTGGTCAGGTGAGCAGCCGGTCATCCAGCC
CTAGTGTCAGAATGATCACTACCTCAGGACCAACCTCAGAGAAGCCAGCTCGCAGTCACCCGTGGACCCCTGATGATT
CCACAGATACCAATGGCTCAGATAACTCCATCCCAATGGCGTATCTTACACTGGATCACCAGCTACAGCCTCTAGCGC
CGTGCCCAAACTCCAAAGAATCCATGGCAGTGTTCGAACAACATTGTAAAATGGCACAGGAGTATATGAAAGTTCAAA
CCGAAATCGCATTGTTACTACAGAGAAAGCAAGAACTAGTTGCAGAATTGGACCAGGATGAAAAGGACCAGCAAAAT
ACATCTCGTCTGGTACAGGAACATAAAAAGCTTTTAGATGAAAACAAAAGCCTTTCTACTTATTACCAGCAATGCAAA
AAACAACTAGAGGTCATCAGAAGCCAACAGCAGAAACGACAAGGCACTTCATGA

The TAK1 deletion mutants comprise a nucleotide sequence homologous to the sequence set forth in SEQ ID Nos: I or 2.

As used herein, the terms"homology","homologue"or"homologous", in any instance, indicate that the nucleic acid sequence referred to, exhibits, in one embodiment at least 70 % correspondence with the indicated sequence. In another embodiment, the nucleic acid sequence exhibits at least 75 % correspondence with the indicated sequence. In another embodiment, the nucleic acid sequence exhibits at least 80 % correspondence with the indicated sequence. In another embodiment, the nucleic acid sequence exhibits at least 85 % correspondence with the indicated sequence. In another embodiment, the nucleic acid sequence exhibits at least 90 % correspondence with the indicated sequence. In another embodiment, the nucleic acid sequence exhibits at least 95 % or more correspondence with the indicated sequence. In another embodiment, the nucleic acid sequence exhibits 95 %-100 % correspondence with the indicated sequence.

Nucleic acid sequence homology may be determined by any number of computer algorithms available and well known to those skilled in the art, for example, the Smith-Waterman algorithm, utilized in analyzing sequence alignment protocols, as in for example, the GAP, BESTFIT, FASTA and TFASTA programs in the Wisconsin Genetics Software Package release 7.0, Genetics Computer Group, 575 Science Dr., Madison, WI). For example, the percent homology between two nucleotide sequences may be determined using the GAP program in the GCG software package, using a NWS gap DNA CMP, matrix and a gap weight of 40, 50, 60,70, or 80 and a length weight of 1,2, 3,4, 5, or 6.

Nucleic acid sequence homology may be determined by hybridization to a reference sequence under highly stringent (0.2xSSC at 65 °C.), stringent (e. g. 4xSSC at 65 °C or 50% formamide and 4xSSC at 42 °C.), or relaxed (4xSSC at 50 °C. or 30-40% formamide and 4xSSC at 42 °C.) conditions.

According to this aspect of the invention, in one embodiment, the oligonucleotide will comprise at least 12 or 15, or in another embodiment, at least 20 or 25, or in another embodiment, at least 30, or in another embodiment, at least 35, or in another embodiment, at least 40, or in another embodiment, at least 45, or in another embodiment, at least 50, or in another embodiment, at least 55, or in another embodiment, at least 60, or in another embodiment, at least 65, or in another embodiment, at least 75 nucleotides in length, specifically hybridizable with the isolated nucleic acid of SEQ ID Nos : 1 or 2.

The oligonucleotide of this invention, according to another embodiment, may be in either sense or antisense orientation, may comprise DNA or RNA, and/or may be single or double stranded. The invention also provides, in other embodiments, compositions and/or vectors comprising the oligonucleotides of this invention.

In one embodiment, the oligonucleotide is in either sense or antisense orientation. In another embodiment, the oligonucleotide is either single or double- stranded. In another embodiment, the invention provides for a vector or composition comprising the oligonucleotides of the invention.

Techniques for introducing the above described recombinant nucleic acids and I vectors, , and others to be described hereinbelow, used in the present invention, are as described, and are to be considered embodiments of the invention for each instance of nucleic acid or vector delivery.

Determination of whether the methods employed disrupt TAK1-SMAD MH2 interaction may be accomplished through a variety of means well known in the art, including, but not limited to chemical cross-linking of the proteins, the yeast two hybrid system, and co-immunoprecipitation.

SMAD signaling pathways have been shown to be essential for bone formation, or osteogenesis. Osteoblast differentiation is SMAD-dependent, mediated via BMP-2 expression. Mesenchymal stem cells, in the presence of recombinantly expressed BMP2 undergo osteogenic differentiation via BMP-SMAD dependent signal transduction cascade.

Further, a method of stimulating or enhancing BMP-mediated SMAD activity comprising the steps of administering an agent that diminishes or abrogates TAK1 expression or function is described herein.

Mesenchymal stem cells engineered to constitutively express BMP2 demonstrate osteoblast differentiation, as compared to cells not expressing BMP2. Cells expressing BMP-2 yet further engineered to express TAK1, however, exhibited a phenotype consistent with mesenchymal stem cells, not expressing BMP2, i. e. TAK1 expression prevented osteogenic differentiation. Expression of the dominant negative TAK1 mutant demonstrated enhanced osteogenic differentiation.

As used herein the term"BMP"refers to the bone morphogenic family of proteins. BMPs bind their cognate receptors in order to exert their effect. Cells found to express both BMP type I and type II receptors include human mesenchymal stem cells (hMSCs).

Examples of members of the BMP family include, but are not limited to: BMP-1, BMP-2, BMP-3, BMP-4, BMP-5, BMP-6, BMP-7, BMP-8, BMP-9, BMP-10 and BMP-11.

The agent employed to diminish or abrogate TAK1 expression or function in BMP-mediated SMAD activity, or for any application in diminishing or abrogating TAK1 function is as defined above, and is to include physical inhibitors to TAK1-SMAD binding, as well as inhibitors of TAK1 expression.

The addition of BMP may precede or accompany the administration of the agent to diminish or abrogate TAK1 expression or function, as a means of enhancing or stimulating BMP-mediated SMAD activity, and represents another embodiment of the present invention.

In another embodiment, the nucleic acids, or vectors comprising an agent to diminish or abrogate TAK1 expression or function may further comprise a second nucleic acid that functions in osteogenesis.

The second nucleic acid may, in one embodiment, correspond to a nucleic acid, encoding the osteogenic factors OP-1, OP-2, BMP-5, BMP-6, BMP-2, BMP-3, BMP-4, BMP-9, DPP, Vg-1, 60A or Vgr-1, each of which represents an embodiment of the invention.

In another embodiment, there is provided a method of enhancing osteogenesis in a subject in need, comprising the use of an agent that mitigates or abrogates TAK1 expression or function to a cell with osteogenic potential in the subject, thereby enhancing osteogenesis in said subject.

In other embodiments, the agent may be administered to the subject via direct delivery to a specific tissue site, such as, for example, by injection or catheterization, or indirectly via parenteral administration, by for example, osmotic pump delivery, aerosol exposure, and numerous methods well described in the art, each of which represents a separate embodiment of the present invention.

In another embodiment, administration can be accomplished in vitro, for example, by direct injection into a cell, or any other means well known to one skilled in the art. Indirect administration may be accomplished in vitro, for example, by supplementing a media surrounding a given cell with an agent, which enables the cell to ultimately be exposed to the agent. Ex-vivo culture of the cells is another embodiment of the invention, whereby a cell is contacted with an agent, a nucleic acid, a vector, a cell or a composition as described herein, then implanted into a subject in need.

In another embodiment, the cell may be loaded on a scaffolding material, prior to its administration to a host. By the term"scaffold"or"scaffolding material"it is meant, in one embodiment, to include a porous structural device that enables cell growth within the device, providing adequate nutrient exchange for cell growth. A scaffold can form a base which serves as a guide for tissue growth.

In one embodiment, the scaffold is formed of a bio-absorbable, or biodegradable, synthetic polymer such as a polyanhydride, polyorthoester, polylactic acid, polyglycolic acid, and copolymers or blends thereof. In another embodiment, non-degradable materials can also be used to form the scaffold. Examples of suitable materials include ethylene vinyl acetate, derivatives of polyvinyl alcohol, teflon, and nylon. In another embodiment, non-degradable materials are a polyvinyl alcohol sponge, or allcylation, and acylation derivatives thereof, including esters. A non-absorbable polyvinyl alcohol sponge is available commercially as Iva-Ion. TM. , from Unipoint Industries. Methods for making this material are described in U. S. Pat. No. 2,609, 347 to Wilson; U. S. Pat. No. 2,653, 917 to Hammon, U. S. Pat. No. 2,659, 935 to Hammon, U. S. Pat. No. 2,664, 366 to Wilson, U. S. Pat. No. 2,664, 367 to Wilson, and U. S. Pat. No. 2,846, 407. In another embodiment, non-biodegradable polymer materials can be used, including polymethacrylate and silicon polymers.

It is to be understood that the terms"administration"or"provision"or "contacting"are meant to be synonymous, and refer to both direct or indirect exposure as described herein, and that any mode of administration utilized for an agent, a nucleic acid, a vector, a cell or a composition as described herein, is to be considered within the framework of envisioned embodiments of this invention.

In, another embodiment of the invention, the agent mitigates or abrogates TAK1 expression or function following TAK1 activation by proinflammatory cytokines.

Inflammation is known to interfere with osteogenesis. As such, it may be desired to promote osteogenesis at a site of inflammation in a subject, including in instances where such inflammation is refractive to control measures, or the measures prove contraindicated for any number of reasons.

In another embodiment, inhibiting or abrogating TAK1 expression or function via the methods and agents described herein will be effective in the presence of any proinflammatory cytokine as well known in the art, including, for example, the pro- inflammatory cytokines IL-1 or TNF-alpha.

According to this aspect of the invention, osteogenesis is promoted via administration of the agent to a cell with osteogenic potential at an inflammatory site, or in another embodiment, via delivery of a cell with osteogenic potential to an inflammatory site.

As used herein the term"cell with osteogenic potential"refers, in one embodiment, to any cell that differentiates, or may be induced to differentiate to a cell that participates in bone deposition.

In another embodiment, a cell with osteogenic potential may be a mesenchymal stem cell, a progenitor cell, an osteoblast, or any cell capable of differentiating into an osteoblast.

In another embodiment of the invention, the subject in need suffers from inflammation-mediated bone loss, and thus stimulation of osteogenesis would serve a therapeutic purpose in the subject.

In another embodiment of the invention, the subject suffering from inflammation-mediated bone loss, may be treated locally at the site of inflammation or systemically. Systemic treatment with an agent downregulating or abrogating TAK1 expression or function may be accompanied by, in still other embodiments, the incorporation of additional moieties that help target the agent to the site of inflammation, including, but not limited to, the use of vector systems that additionally encode for targeting molecules including integrins, by methods well known in the art.

In another embodiment, the subject in need according to this aspect of the invention, suffers from periodontal disease, osteoarthritis, Köhler's bone disease, rheumatoid arthritis or osteoporosis. These diseases are not meant to be limiting however, and the methods, nucleic acids, vectors and compositions disclosed herein for promotion of osteogenesis may also find utility for subjects suffering from other diseases associated bone loss, including for example, some forms of osteomyelitis, and osteosarcoma. Each of these applications is to be considered an additional embodiment of the present invention.

Osteogenesis is marked by the differentiation of progenitor osteoblasts, whose differentiation heralds their effector function in bone induction.

In one embodiment, osteogenesis stimulated or enhanced by the methods, agents, nucleic acids, vectors and/or compositions described herein may be measured via assessment of cell surface expression of osteopontin and BSP-II, which may be determined by FACS analysis, immuno-histochemistry or immunofluorescence assay. Osteogenesis, in other embodiments, may be determined via assaying cell alkaline phosphatase (ALP) activity by known histological techniques, or via assaying interleukin-6 (IL-6) and osteocalcin gene expression via methods well known in the art such as RT-PCR, Northern blot analysis or RNase protection assay of via assaying protein levels via Western blot analysis, ELISA or RIA.

Stimulation or enhancement of osteogenesis is, in one embodiment, beneficial not only in cases of bone loss, but in bone damage, as well. Such damage may be a result of any number of conditions, including, but not limited to bone fracture, genetic diseases, kidney disease, some infections or inflammation.

It is to be understood that the agent mitigating or abrogating TAK1 expression delivered to the subject may comprise any of the embodiments listed above, and the agents may comprise any of the embodiments listed herein, that result in the production of enhanced bone repair as a result of enhanced osteogenesis.

Changes in bone volume, quality or strength as a function of bone repair may be measured by a number of methodologies well known to one skilled in the art, including methods directly measuring tensile strength, and methods measuring various bone markers, as described in US Patent No. 5, 785, 041 by Weinstein et al. , US Patent No. 5,509, 042 by Mazess et al. , Ronis M. J. J. et al Toxicol Sci, (2001) 62: 321-329 or Suponitsky I. et al Journal of Endocrinology (1998) 156: 51-57. Bone repair may be taken as an improvement in measurements as above, as a function of the methods listed herein.

The following examples further illustrate some aspects of the invention herein described. These examples, combined with the specification hereinabove, are intended for exemplification purposes, and are not to be construed as a means of limiting the present invention.

## Claims

1. Use of an agent which is a nucleic acid that mitigates or abrogates TAK1 expression or function by endogenous TAK1 sequence inactivation for the manufacture of a medicament for enhancing osteogenesis and/or bone repair wherein i) the nucleic acid is an antisense RNA or ii) a synthetic oligonucleotide capable of hybridising with TAK1 double stranded DNA to form a triple helix, or iii) the nucleic acid agent comprises an siRNA, said siRNA may consist of 15 to 30 nucleotides.

2. The use of claim 1 wherein said medicament is for treating a disease selected from the group consisting of inflammation-mediated bone loss, periodontal disease, osteoarthritis, Kohler's bone disease, rheumatoid arthritis and osteoporosis, in particular, rheumatoid arthritis.

## Patentansprüche

1. Verwendung eines Mittels, das eine Nukleinsäure ist, die die TAK1 Expression oder Funktion durch Inaktivierung der endogenen TAK1 Sequenz abschwächt oder unterbricht zur Herstellung eines Medikaments zur Verstärkung der Osteogenese und/oder der Knochenreparatur, wobei i) die Nukleinsäure eine Antisense RNA ist oder ii) ein synthetisches Oligonukleotid, das mit der TAK1 Doppelstrang DNA hybridisieren kann, um eine Tripplehelix zu bilden oder iii) das Mittel einer Nukleinsäure eine siRNA umfasst, diese siRNA kann aus 15 bis 30 Nukleotiden bestehen.

2. Verwendung nach Anspruch 1, wobei das Medikament zur Behandlung einer Erkrankung ausgewählt aus der Gruppe bestehend aus entzündungsvermittelten Knochenverlust, periodontaler Erkrankung, Osteoarthritis, Kohler's Knochenerkrankung, rheumatoider Arthritis und Osteoporose insbesondere, rheumatoider Arthritis.

## Revendications

1. Utilisation d'un agent qui est un acide nucléique qui atténue ou abolit l'expression ou le fonctionnement de TAK1 par l'inactivation de la séquence de TAK1 endogène pour la fabrication d'un médicament destiné à améliorer l'ostéogénèse et/ou la réparation osseuse, **caractérisée en ce que** i) l'acide nucléique est un ARN antisens ou ii) un oligonucléotide de synthèse capable de s'hybrider avec l'ADN double brin de TAK1 pour former une triple hélice, ou iii) l'agent d'acide nucléique comprend un ARNsi, ledit ARNsi peut être constitué de 15 à 30 nucléotides.

2. Utilisation selon la revendication 1, pouvant que ledit médicament est destiné au traitement d'une maladie choisie dans le groupe constitué de la perte osseuse médiée par une inflammation, la maladie parodontale, l'ostéoarthrite, la maladie de Köhler, la polyarthrite rhumatoïde et l'ostéoporose, en particulier la polyarthrite rhumatoïde.
